Europäisches Patentamt

European Patent Office

Office européen des brevets

(19)

(11) **EP 1 280 732 B1**

(12) **FASCICULE DE BREVET EUROPEEN**

(45) Date de publication et mention
de la délivrance du brevet:
**02.06.2004 Bulletin 2004/23**

(51) Int Cl.[7]: **C01B 25/32**, A61L 27/12,
A61L 27/32

(21) Numéro de dépôt: **01916835.0**

(22) Date de dépôt: **09.04.2001**

(86) Numéro de dépôt international:
**PCT/CH2001/000224**

(87) Numéro de publication internationale:
**WO 2001/081243 (01.11.2001 Gazette 2001/44)**

(54) **MICROGRANULES PHOSPHOCALCIQUES**

KALZIUMPHOSPHATMIKROGRANULATE

CALCIUM PHOSPHATE MICROGRANULES

(84) Etats contractants désignés:
**AT BE CH CY DE DK ES FI FR GB GR IE IT LI LU
MC NL PT SE TR**

(30) Priorité: **26.04.2000 CH 814002000**

(43) Date de publication de la demande:
**05.02.2003 Bulletin 2003/06**

(73) Titulaires:
• **Ecole Polytechnique Fédérale de Lausanne
1015 Lausanne (CH)**
• **Lemaître, Jacques
1015 Lausanne (CH)**

(72) Inventeur: **TERRAZZONI, Stéphane
CH-1110 Morges (CH)**

(74) Mandataire: **Besse, François
ANDRE ROLAND IPS,
15, Avenue Tissot,
P.O. Box 1255
1001 Lausanne (CH)**

(56) Documents cités:
**US-A- 3 384 451**

• **DATABASE WPI Section Ch, Week 199034
Derwent Publications Ltd., London, GB; Class
B04, AN 1990-257489 XP002142907 & JP 02
180709 A (TONEN CORP), 13 juillet 1990
(1990-07-13)**
• **PAUL W ET AL: "Development of porous
spherical hydroxyapatite granules: application
towards protein delivery" J MATER SCI MATER
MED;JOURNAL OF MATERIALS SCIENCE:
MATERIALS IN MEDICINE 1999 KLUWER
ACADEMIC PUBLISHERS, DORDRECHT,
NETHERLANDS, vol. 10, no. 7, 1999, pages
383-388, XP002142926**
• **DATABASE WPI Section Ch, Week 199110
Derwent Publications Ltd., London, GB; Class
D22, AN 1991-068944 XP002142913 & JP 03
016906 A (NIPPON CHEM IND CO LTD), 24 janvier
1991 (1991-01-24)**
• **DATABASE WPI Section Ch, Week 199034
Derwent Publications Ltd., London, GB; Class
B04, AN 1990-257487 XP002142914 & JP 02
180707 A (TONEN CORP), 13 juillet 1990
(1990-07-13)**
• **DATABASE WPI Section Ch, Week 199120
Derwent Publications Ltd., London, GB; Class
A97, AN 1991-146055 XP002142915 & JP 03
083805 A (SUMITOMO CHEM IND KK), 9 avril
1991 (1991-04-09)**

**Description**

Domaine de l'invention

La présente invention concerne des microgranules réalisés à partir de matériaux phosphocalciques.

L'invention concerne également un procédé de production de ces microgranules.

Les microgranules selon l'invention peuvent être utilisés pour de nombreuses applications dans le domaine biomédical (matériaux de comblements osseux, revêtements d'implants métalliques par plasma spray, supports de culture de cellules osseuses ou excipients pour principes actifs) en raison de leur biocompatibilité et de leurs caractéristiques contrôlables de manière indépendante (composition, taille, distribution granulométrique, porosité, état de surface). Ils peuvent également servir en chromatographie en phase liquide.

État de la technique

Les phosphates de calcium, en particulier l'hydroxyapatite et le phosphate tricalcique ß, sont connus pour être utilisés dans de nombreuses applications biomédicales en raison de leur composition chimique proche de la phase minérale de l'os ce qui les rend parfaitement biocompatibles. De plus, contrairement à des biomatériaux inertes comme l'alumine ou la zircone, ils sont bioactifs et ostéoconducteurs c'est-à-dire qu'ils favorisent les échanges entre les cellules et le tissu receveur ainsi que l'ostéogenèse.

En chirurgie orthopédique ou maxillo-faciale, lorsqu'il s'agit de renforcer ou de remplacer du tissu osseux manquant à la suite d'une maladie ou d'un accident, ces matériaux se présentent souvent sous forme de granules plus ou moins sphériques. Sous cette forme, ils sont incorporés à des ciments phosphocalciques injectables, à des solutions aqueuses contenant des polymères dérivées de cellulose (US 5,717,006), à du sang (US 5,064,436) ou servir à la réalisation d'implants céramiques préformés ex-vivo. Mélangés à des ciments de type PMMA, ils sont employés pour le scellement de prothèse et comme matériaux de comblement osseux (US 4,842,603). Les granules de phosphate de calcium sont également utilisés en chirurgie dentaire en présence de liants biocompatibles par exemple, pour l'augmentation de la paroi alvéolaire, le comblement de site après extraction de racine ou l'augmentation des tissus durs (US 5,702,677, ou Hench (1991) J. Am. Cer. Soc cf.). Dans ces domaines d'application, les granules phosphocalciques employés ont des diamètres allant de quelques microns à 2-3 millimètres. Par ailleurs, il a été mis en évidence que ces granules doivent être de préférence sphérique et avoir un bon état de surface afin de prévenir toutes réactions inflammatoires se déclarant souvent avec des pièces implantées de forme coupante ou anguleuse. Dans le cas des ciments phosphocalciques ou PMMA injectables, les caractéristiques requises sont des tailles n'excédant pas 200-300 µm, une distribution granulométrique étroite et des formes les plus sphériques possibles afin de garantir de bonnes caractéristiques rhéologiques à la pâte injectée. Il est également possible de jouer sur la porosité pour contrôler la vitesse de résorption du matériau implanté en milieu biologique ; à composition égale, plus la fraction volumique poreuse est importante, plus le granule aura tendance à disparaître rapidement. De même, pour éviter tout phénomène d'encapsulation apparaissant essentiellement pour les pièces de grandes tailles, il est nécessaire d'avoir une porosité homogène et si possible interconnectée afin de faciliter les échanges et la circulation des liquides physiologiques et des cellules, en particulier les macrophages (US 5,034,352, US 5,064,436). Par contre, pour des sites receveurs sollicités mécaniquement, une porosité trop importante n'est pas souhaitée car elle entraîne un trop grand affaiblissement du granule implanté.

Les granules de phosphates de calcium sont aussi employés pour le revêtement d'implants métalliques par plasma spray, comme supports de culture de cellules osseuses in vitro (US 4,457,017) ou encore comme excipients pour le relâchement contrôlé de principes actifs de comprimé pharmaceutique. Là encore, les poudres granulées doivent avoir des caractéristiques bien adaptées à chaque cas. Par exemple, les procédés de déposition par plasma spray exigent : d'une part des poudres quasi-monodisperses afin d'améliorer le rendement de déposition ; d'autre part de bonnes propriétés de coulabilité pour faciliter le transport des poudres à déposer. En ce qui concerne les excipients pour comprimés pharmaceutiques, il est important que les caractéristiques soient identiques pour l'ensemble des granules. En effet, la vitesse de relâchement des principes actifs dépend étroitement de la forme, de la taille, de la surface spécifique et de la porosité (US 5,066,441 et US 5,055,307). Une variation de l'une de ces caractéristiques entraîne donc un moins bon contrôle de la vitesse de relâchement.

Les phosphates de calcium, en particulier les particules d'hydroxyapatite, sont aussi utilisés en chromatographie en phase liquide en raison de leur bonne sélectivité d'absorption. Ils permettent la séparation et la purification de biopolymères, protéines, enzymes, monosaccharides et de l'eau (US 5,217,699 - US 5,158,756 - Re 35,340). Les caractéristiques recherchées pour ces granules sont d'une part ; des formes sphériques et des tailles régulières afin de remplir de manière uniforme et avec une densité homogène les colonnes de séparation ; d'autre part de bonne résistance mécanique et des diamètres de l'ordre de 50-100 µm pour permettre un flux rapide des phases mobiles tout en évitant des problèmes d'encrassement de la colonne en particulier en HPLC (High Performance Liquid Chromatography). De plus, les granules doivent posséder une microstructure finement divisée et uniforme (porosité fermée et ouverte) pour améliorer leur potentiel de sélectivité.

Les poudres phosphocalciques granulées présentent cependant l'inconvénient d'avoir des caractéristiques qui ne répondent que partiellement au cahier des charges mis en place dans ces différents domaines d'applications. Tout d'abord, la majorité des granules disponibles dans des tailles de l'ordre de 50-1000 μm est réalisée par simple broyage-classification à partir de blocs céramiques frittés mises en forme par pressage à sec ou par coulage en présence d'un liant. Les granules obtenus, bien que de porosité contrôlable, ont des formes non définies, souvent angulaires, ce qui augmente les risques d'inflammation dans les applications médicales (comblement osseux,...). Pour les mêmes raisons, leur utilisation comme excipient pour le relâchement de principes actifs ou en chromatographie n'est pas appropriée. Ce procédé n'autorise pas non plus une distribution granulométrique étroite préférable pour un meilleur contrôle des durés de résorption en milieu biologique ou encore l'amélioration des caractéristiques rhéologiques des pâtes dans le cas des ciments injectables. Des étapes supplémentaires de classification et de polissage, longues et coûteuses, sont donc souvent nécessaires.

Il est également possible de produire des granules de phosphate de calcium dans les mêmes gammes de taille par simple agglomération de poudres fines à l'aide de liants organiques ou d'eau. Pour ce faire, le liant est introduit de manière contrôlée dans un lit fluidisé de poudre fine (US 5,034,352) ou encore dans un tambour rotatif dans lequel est brassée la poudre (US 5,702,677). Les granules ainsi obtenus sont de forme sphérique avec un bon état de surface. En revanche, leur distribution granulométrique est étalée et il s'avère difficile d'obtenir des densités élevées.

D'autres techniques bien connues dans le domaine des céramiques, comme la granulation par séchage-atomisation de suspensions chargées en poudre fine, permettent de produire des granules sphériques dans des gammes de tailles inférieures à celles précitées, par exemple de l'ordre de 10-100 μm. Par contre, ces granules sont souvent creux, de distribution granulométrique large et de diamètre médian difficilement supérieur à 100 μm sans avoir recours à l'utilisation d'une tour de séchage trop imposante.

Ainsi, les granules à base de phosphate de calcium de l'état de la technique et leurs procédés de production présentent plusieurs inconvénients. En particulier, ces techniques sont peu flexibles, et de ce fait, il est difficile d'obtenir des granules dont les caractéristiques sont parfaitement contrôlées de manière indépendante.

Description de l'invention

La présente invention a notamment le mérite de remédier aux problèmes susmentionnés. Elle se rapporte à des microgranules phosphocalciques obtenus selon les modalités telles que définies dans la revendication 1 et dont le rapport atomique Ca/P est compris entre 1 et 2 et qui se caractérisent par le fait que chaque microgranule a sensiblement la forme d'un ellipsoïde de révolution de grand axe a et de petit axe b et que la distribution granulométrique est telle que la déviation standard par rapport à la longueur moyenne de l'un quelconque des deux axes **a** ou **b** est inférieure à 5 %.

L'invention concerne également un procédé permettant leur production. Avec la présente invention, les microgranules sont contrôlables à volonté et de manière indépendante au niveau de leur composition, forme, diamètre médian, distribution granulométrique et de leur porosité.

Outre leur distribution granulométrique pouvant être extrêmement étroite, centrée de préférence autour d'un diamètre médian compris entre 50 μm et 3000 μm, les microgranules selon l'invention présentent une porosité uniformément répartie, interconnectée ou non, occupant une fraction volumique comprise entre 0 et 70 % du volume total apparent.

La granulation se fait à partir d'une suspension chargée de poudre de phosphate de calcium et/ou d'autres sels de calcium peu solubles, auxquelles peuvent s'ajouter divers additifs minéraux ou organiques (déflocculants, plastifiants, liants, porogènes). La suspension peut également contenir un ou plusieurs agents gélifiants. Un soin particulier est apporté lors de la préparation de la suspension afin d'obtenir les propriétés rhéologiques nécessaires pour l'utilisation du procédé de granulation durant lequel la suspension est mise en perturbation pour être transformée en gouttelettes. Les gouttelettes sont ensuite préconsolidées, par exemple par réaction chimique, en présence d'une solution ionique, puis lavées et séchées. L'étape de consolidation finale peut se faire soit par frittage dans le cas de microgranules céramiques à des températures comprises entre 900°C et 1600°C, soit chimiquement dans le cas de microgranules en matériaux cimentaires ; la réaction chimique se déroulant en atmosphère plus ou moins humide et à des températures pouvant varier de préférence entre 0°C et 250°C.

Selon le cas, ils se présentent soit sous forme de matériaux céramiques consolidés par frittage soit sous forme de matériaux cimentaires consolidés chimiquement. Les microgranules céramiques peuvent être, par exemple en hydroxyapatite, en phosphate tricalcique-β ou encore en un mélange des deux. Les microgranules cimentaires peuvent être obtenus à partir d'un mélange en milieu aqueux de deux poudres coréactives, l'une acide et l'autre basique, qui provoque la réaction de dissolution des deux réactifs et la précipitation d'une nouvelle phase thermodynamiquement plus stable. Dans le cadre de cette invention, diverses formulations connues de ciments phosphocalciques peuvent être envisagées. En particulier, un nouveau ciment hydroxyapatite, obtenu par étuvage à 121 °C en atmosphère saturée d'humidité, d'un mélange de monétite (phosphate dicalcique anhydre) et de carbonate de calcium (calcite, aragonite ou vatérite) convient spécialement.

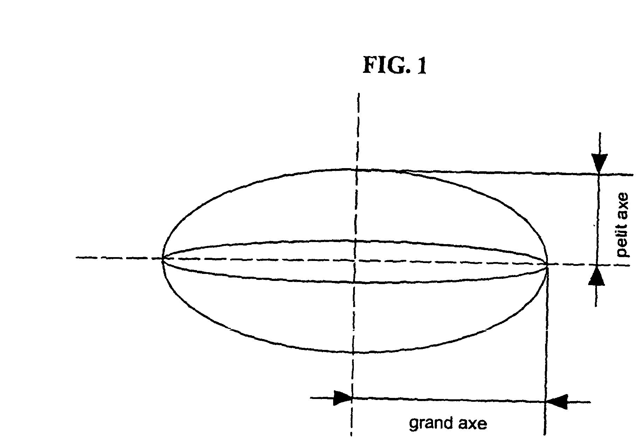

La forme des microgranules correspond à un ellipsoïde de révolution pouvant être plus ou moins aplati par exemple sphérique avec une définition très précise à plus ou moins 5 % du facteur de forme lui-même compris entre 1 et 3. Selon l'invention, le terme "facteur de forme" désigne le rapport entre le grand axe et le petit axe d'un ellipsoïde de révolution (voir Fig. 1). Dans le cas de forme sphérique correspondant à un facteur de forme égale à 1, la distribution granulométrique peut être extrêmement étroite avec une déviation standard ne dépassant pas 2 % autour d'un diamètre médian compris entre 50 µm et 3000 µm, de préférence entre 100 µm et 2000 µm. La structure poreuse des microgranules, correspondant à la fraction volumique poreuse occupée, le diamètre moyen des pores et le degré d'interconnection, est contrôlable à volonté. Ainsi, la porosité des microgranules est uniformément répartie et peut être selon le cas, interconnectée ou non. Elle est parfaitement contrôlable en ce sens qu'il est possible de faire varier la fraction volumique poreuse de 0 à 70 % du volume total apparent, la taille des pores de 0.5 µm à 100 µm, de préférence de 1 µm à 10 µm, ainsi que la taille des interconnections entre pores dans le cas de porosité interconnectée. Par exemple, des paramètres comme la fraction volumique de poudre dans le suspension phosphocalcique à granuler, l'utilisation de phase porogène dans cette même suspension ou encore le traitement de consolidation final (chimique ou par frittage) permettent l'obtention de la structure poreuse désirée.

La réalisation de la suspension contenant la ou les poudres phosphocalciques fait l'objet d'un soin particulier afin d'obtenir des propriétés rhéologiques nécessaires pour l'utilisation du procédé de granulation. Selon le cas, la rhéologie et l'homogénéité de la suspension peuvent être optimisées par addition de polymères hydrosolubles, de préférence biocompatibles dans le cas de microgranules cimentaires (acide polyacrylique, etc...). Différents traitements sont également utilisés pour broyer ou désagglomérer les poudres afin de mieux disperser et ainsi homogénéiser la suspension.

L'invention concerne également un procédé de production de ces microgranules à partir d'une suspension chargée en poudre de phosphate de calcium et/ou d'autres sels de calcium peu solubles, auxquelles peuvent s'ajouter divers additifs minéraux ou organiques (défloculants, plastifiants, liants, porogènes) ainsi que différents types d'agents gélifiants. Le procédé consiste en ce que la suspension passe au travers d'une buse de diamètre défini puis est convertie en gouttelettes par perturbation. Les gouttelettes ainsi formées sont préconsolidées dans une solution ionique par réaction chimique, lavées et séchées. Selon l'invention, le terme "solution ionique" correspond à une solution contenant des ions qui se combinent avec "l'agent gélifiant" contenu dans la suspension pour former un composé très peu soluble. Dans le cadre de l'invention, divers couples agent gélifiant-ion sont envisageables par exemple l'alginate avec des ions $Ca^{2+}$ ou $Mg^{2+}$, l'acide polyvinylique avec des ions borates ou de l'ammoniaque, etc...Contrairement aux procédés d'atomisation de l'état de l'art, la suspension n'est pas à proprement parler pulvérisée. Elle sort de la buse sous forme d'un jet laminaire qui, sous l'action d'une perturbation, est segmenté en fines gouttelettes. Selon l'invention, la perturbation peut être induite par vibrations mécaniques, électromagnétiques, pneumatiques, piézoélectriques ou encore par irradiation électroacoustique; elle peut être appliquée à la buse et/ou au système d'alimentation de la suspension et/ou au réservoir contenant la suspension. En outre, il est essentiel que la perturbation se produise à fréquence constante, de préférence entre 50 et 20.000Hz, afin de garantir une segmentation constante de la suspension et ainsi la production de gouttelettes de volume quasi identique. Les gouttelettes tombent ensuite le long d'une colonne de hauteur variable en prenant, en cours de chute, une forme plus ou moins sphérique sous l'effet de la force de tension de surface. Enfin, les gouttelettes sont récupérées dans une solution ionique pour y être préconsolidées par réaction chimique. Selon le procédé de l'invention, un courant généré au sein de la solution ionique permet d'ajuster la durée de séjour des gouttelettes dans la solution et ainsi de contrôler de manière reproductible le degré de préconsolidation. Ce courant permet également d'éliminer tout risque de déformation par collision des gouttelettes entre elles ; les gouttelettes étant entraînées au fur et à mesure de leur production.

Divers dispositifs permettent de réduire les déformations des gouttelettes pouvant survenir lors de " l'impact " de ces dernières avec la surface de la solution ionique. Par exemple, les gouttelettes peuvent être ralenties en tombant au travers d'une couche de mousse générée à la surface de la solution, d'épaisseur comprise entre 5 mm et 100 mm, de préférence entre 5 mm et 50 mm. D'autre part, l'ajout de surfactant ou de solvant organique, par exemple des alcools comme l'éthanol, le propanol, etc., permet de diminuer la tension de surface de la solution ionique. Selon un mode préférentiel de l'invention, la couche de mousse à la surface de la solution peut être obtenue à partir de solution à base de surfactants ou de solvants organiques.

Selon le procédé de l'invention, la viscosité de la suspension phosphocalcique doit être inférieure à 200 mPa. s ; le diamètre de la buse est compris entre 50 µm et 3000 µm.

Les microgranules et le procédé permettant leur production présentent de multiples avantages. En particulier, les microgranules selon l'invention se caractérisent par le fait que leurs caractéristiques (composition, forme, diamètre médian, distribution granulométrique, porosité) sont parfaitement contrôlables et de manière indépendante.

Les microgranules à base de phosphate de calcium peuvent être utilisés pour de nombreuses applications dans le domaine biomédical en raison de leur biocompatibilité et de leurs caractéristiques contrôlables de manière indépendante. Selon l'invention, ces microgranules sont réalisés à partir de phosphate de calcium dont le rapport atomique est compris entre 1 et 2. Elles se présentent soit sous forme de matériaux céramiques consolidés par frittage soit sous forme de matériaux cimentaires consolidés chimiquement. Ces microgranules ont d'une part; une forme correspondant à une ellipsoïde de révolution plus ou moins aplati, par exemple sphérique, avec un facteur de forme défini à plus ou moins 5 % entre 1 et 3; d'autre part une distribution granulométrique pouvant être extrêmement étroite avec, dans le cas de granules sphériques, une déviation standard ne dépassant pas 2 % autour d'un diamètre médian compris entre 50 μm et 3000 μm, de préférence entre 100 μm et 2000 μm. La porosité des microgranules est uniformément répartie et peut être selon le cas interconnectée ou non. Elle est parfaitement contrôlable en ce sens qu'il est possible de faire varier la fraction volumique poreuse de 0 % à 70 % du volume total apparent, la taille des pores de 0.5 μm à 100 μm, de préférence de 1 μm à 10 μm, ainsi que la taille des interconnections entre pores dans le cas de porosité interconnectée. Enfin, pour les microgranules réalisés à partie de matériaux cimentaires, le mode de consolidation chimique confère au granule un caractère hautement bioactif supérieur à celui rencontré pour des compositions similaires consolidées par frittage à haute température. Les microgranules conservent ainsi une porosité importante qui favorise la biorésorption et permet leur imprégnation avec des principes actifs.

Ainsi et selon l'état de la technique décrit précédemment, ces microgranules incorporés à des solutions aqueuses contenant des polymères dérivées de cellulose, du sang ou des ciments injectables de types phosphocalciques ou PMMA, peuvent servir comme matériaux de comblement et de renforcement osseux ou encore pour le scellement de prothèse. Elles peuvent être aussi employées pour le revêtement d'implants métalliques par plasma spray, comme supports de culture de cellules osseuses in vitro ou encore comme excipients pour le relâchement contrôlé de principes actifs de comprimé pharmaceutique. Enfin, ces microgranules peuvent être utilisés en chromatographie en phase liquide.

Dans le texte qui suit, l'invention sera illustrée au moyen de quelques exemples:

EXEMPLE 1 : MICROGRANULES CERAMIQUES EN PHOSPHATE TRICALCIQUE β

Une suspension aqueuse composée de poudre de phosphate tricalcique β (β-TCP), d'acide polyacrylique (PAA) et d'alginate de sodium (Na-alginate) est utilisée pour la production de microgranules de phosphate tricalcique-β. Le pH de la suspension est ajusté entre 8-9 par ajout d'ammoniaque. Chaque litre de cette suspension contient :

  1152g de poudre de β-TCP
  3.13g de PAA
  24.2g d'alginate de sodium
  593g d'eau déminéralisée

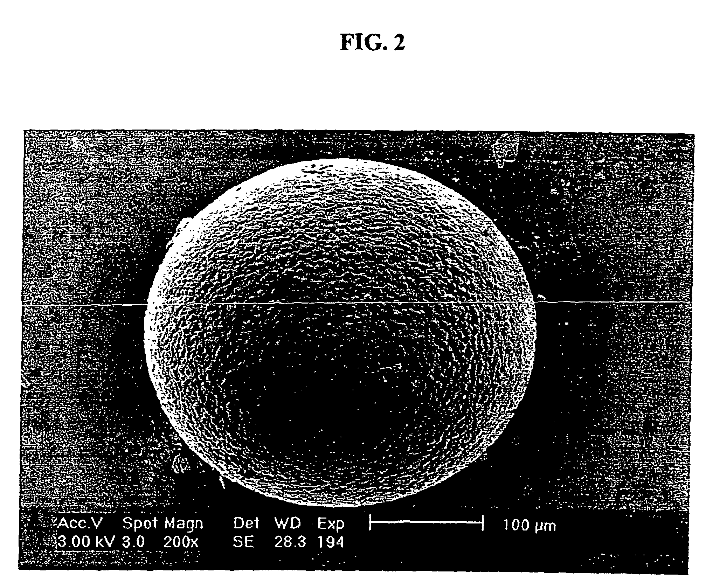

La suspension passe au travers d'une buse de 200 μm de diamètre, vibrée à une fréquence de 2000 Hz à 3000 Hz. Les gouttelettes ainsi formées tombent le long d'une colonne en prenant au cours de leur chute une forme sphérique. Les gouttelettes sont récupérées dans une solution aqueuse de $CaCl_2$ à 2 %pds pour y être préconsolidées pendant 30 minutes sous forme de microbilles. Les microbilles sont ensuite lavées dans de l'eau puis séchées dans un évaporateur rotatif à 60°C. Leur diamètre est égale à 400μm avec une déviation standard de 1-2 %. L'étape suivante consiste en un traitement de densification des microbilles par frittage à 1230°C durant 10 heures dans un four à moufle. A l'issue de ce traitement, les microbilles ont un diamètre de 300μm avec une déviation standard de 1-2 % (voir Fig. 2). Leur densité apparente est égale à 3.00 g/cm$^3$ ce qui correspond à environ 98% de la densité théorique du β-TCP.

EXEMPLE 2: MICROGRANULES CERAMIQUES EN HYDROXYAPATITE

Une suspension aqueuse composée de poudre d'hydroxyapatite (HAp), d'acide polyacrylique (PAA) et d'alginate de sodium (Na-alginate) est utilisée pour la production de microgranules d'hydroxyapatite. Le pH de la suspension est ajusté entre 8-9 par ajout d'ammoniaque. Chaque litre de cette suspension contient :

  1087g de poudre d'HAp
  4.04g de PAA
  5.225g d'alginate de sodium
  644g d'eau déminéralisée

La suspension passe au travers d'une buse de 350μm de diamètre, vibrée à une fréquence de 1000 Hz à 1500 Hz. Comme dans l'exemple 1, les gouttelettes sont récupérées dans une solution aqueuse de $CaCl_2$ à 2 %pds pour y être préconsolidées pendant 30 minutes sous forme de microbilles. Les microbilles sont ensuite lavées dans de l'eau puis séchées dans un évaporateur rotatif à 60°C. Leur diamètre est de 700 μm avec une déviation standard de 1-2 %. L'étape de densification par frittage consiste en un palier de 10 heures à 1270°C dans un four à moufle. Les caractéristiques finales des microbilles sont un diamètre de 600 μm avec une déviation standard de 1-2 % et une densité apparente égale à 3.07 g/cm$^3$ ce qui correspond à environ 97-98 % de la densité théorique de l'hydroxyapatite.

EXEMPLE 3 MICROGRANULES EN CIMENT D'HYDROXYAPATITE

Une suspension composée d'un mélange stoechiométrique de poudres de phosphate dicalcique anhydre (DCP) et de calcite (CC) en présence d'une solution aqueuse contenant de l'acide polyacrylique (PAA) et de l'alginate de sodium (Na-alginate) est utilisée pour la production de microgranules en ciment d'hydroxyapatite. Afin d'amorcer la réaction de cimentation finale, des germes d'hydroxyapatite (HAp) sont également introduits. Le pH de la suspension est ajusté entre 8-9 par ajout d'ammoniaque. Chaque litre de cette suspension contient :

    532.9g de poudre DCP
    261.3g de poudre CC
    130.1g de poudre HAp
    13.4g de PAA
    5.5g d'alginate de sodium
    661.8g d'eau déminéralisée

La suspension passe au travers d'une buse de 150 μm de diamètre, vibrée à une fréquence de 2000 Hz à 3000 Hz. Comme pour les exemples 1 et 2, les gouttelettes sont récupérées dans une solution aqueuse de CaCl$_2$ à 2 %pds pour y être préconsolidées pendant 30 minutes sous forme de microbilles. Les microbilles sont ensuite lavées dans de l'eau puis séchées sous air sec. Leur diamètre est de 400 μm avec une déviation standard de 1-2 %. Les microbilles sont ensuite transformées en hydroxyapatite en autoclave. La consolidation se fait à 121°C pendant 1 heure et conduit à la formation d'hydroxyapatite accompagnée d'un dégagement de gaz carbonique et d'eau :

$$3CaHPO_4 + 2CaCO_3 \rightarrow Ca_5(PO_4)_3OH \downarrow + 2CO_2 \uparrow + H_2O$$

$$DCP + CC \rightarrow HAp$$

Lors de la consolidation, le diamètre des microbilles reste inchangé à 400μm avec une déviation standard de 1-2 %. La densité apparente est de l'ordre de 1.75 g/cm$^3$ soit 55-56 % de la densité théorique de l'hydroxyapatite.

**Revendications**

1. Microgranules phosphocalciques obtenus en effectuant les étapes suivantes :

    - préparation d'une suspension chargée en poudre de phosphate de calcium et comprenant optionnellement au moins un autre sel de calcium peu soluble,
    - ajout optionnel d'au moins un additif minéral ou organique,
    - ajout optionnel d'au moins un agent gélifiant,
    - passage de la suspension au travers d'une buse,
    - sortie de la buse sous forme de jet laminaire,
    - perturbation du jet laminaire de manière à le segmenter en fines gouttelettes,
    - consolidation des gouttelettes,

chaque microgranule ainsi formé ayant sensiblement la forme d'un ellipsoïde de révolution de grand axe a et de petit axe b, l'ensemble des microgranules présentant une distribution granulométrique telle que la déviation standard par rapport à la longueur moyenne de l'un quelconque des deux axes a ou b est inférieure à 5 %.

2. Microgranules selon la revendication précédente, **caractérisés par le fait que** le rapport entre les axes a et b varie entre 1 et 3.

3. Microgranules selon la revendication 2, **caractérisés par le fait que** les deux axes ont la même longueur (rapport entre les axes a et b vaut 1).

**4.** Microgranules selon la revendication précédente, **caractérisés par le fait que** la déviation standard par rapport à la longueur moyenne des axes est inférieure à 2 %.

**5.** Microgranules selon l'une quelconque des revendications précédentes, **caractérisés par le fait qu'**ils présentent une porosité uniformément répartie occupant une fraction volumique comprise entre 0 et 70 % du volume total apparent.

**6.** Procédé de fabrication de microgranules phosphocalciques de volume quasi identique **caractérisé par** les étapes suivantes :

- préparation d'une suspension chargée en poudre de phosphate de calcium et comprenant optionnellement au moins un autre sel de calcium peu soluble,
- ajout optionnel d'au moins un additif minéral ou organique,
- ajout optionnel d'au moins un agent gélifiant,
- passage de la suspension au travers d'une buse,
- sortie de la buse sous forme de jet laminaire,
- perturbation du jet laminaire de manière à le segmenter en fines gouttelettes,
- consolidation des gouttelettes.

### Patentansprüche

**1.** Calciumphosphat-Mikrogranula, die mittels Durchführen der folgenden Schritte erhalten werden:

- Herstellung einer mit Calciumphosphatpulver angereicherten Suspension, die gegebenenfalls mindestens ein weiteres wenig lösliches Calciumsalz umfasst,
- gegebenenfalls Zugabe mindestens eines mineralischen oder organischen Hilfsstoffs,
- gegebenenfalls Zugabe mindestens eines Geliermittels,
- Hindurchleiten der Suspension durch eine Düse,
- Austritt aus der Düse in Form eines Flachstrahls,
- Perturbation des Flachstrahls, so dass er in feine Tröpfchen zerteilt wird,
- Verfestigung der Tröpfchen,

wobei jedes so hergestellte Mikrogranulum im Wesentlichen die Form eines Umdrehungsellipsoids mit der großen Achse a und der kleinen Achse b hat, wobei die gesamten Mikrogranula eine derartige granulometrische Verteilung aufweisen, dass die Standardabweichung, bezogen auf die mittlere Länge einer der beiden Achsen a oder b, kleiner als 5% ist.

**2.** Mikrogranula nach dem vorhergehenden Anspruch, **gekennzeichnet durch** die Tatsache, dass das Verhältnis zwischen den Achsen a und b zwischen 1 und 3 variiert.

**3.** Mikrogranula nach Anspruch 2, **gekennzeichnet durch** die Tatsache, dass die beiden Achsen die gleiche Länge haben (Verhältnis zwischen den Achsen a und b ist 1) .

**4.** Mikrogranula nach dem vorhergehenden Anspruch, **gekennzeichnet durch** die Tatsache, dass die Standardabweichung, bezogen auf die mittlere Länge der Achsen kleiner als 2% ist.

**5.** Mikrogranula nach einem der vorhergehenden Ansprüche, **gekennzeichnet durch** die Tatsache, dass sie eine gleichmäßig verteilte Porosität aufweisen, die eine Volumenfraktion zwischen 0 und 70%, bezogen auf das apparente Gesamtvolumen, einnimmt.

**6.** Verfahren zur Herstellung von Calciumphosphat-Mikrogranula mit quasiidentischem Volumen, das durch die folgenden Schritte gekennzeichnet ist:

- Herstellung einer mit Calciumphosphatpulver angereicherten Suspension, die gegebenenfalls mindestens ein weiteres wenig lösliches Calciumsalz umfasst,

- gegebenenfalls Zugabe mindestens eines mineralischen oder organischen Hilfsstoffs,
- gegebenenfalls Zugabe mindestens eines Geliermittels,
- Hindurchleiten der Suspension durch eine Düse,
- Austritt aus der Düse in Form eines Flachstrahls,
- Perturbation des Flachstrahls, so dass er in feine Tröpfchen zerteilt wird,
- Verfestigung der Tröpfchen.


**Claims**

1. Calcium phosphate microgranules obtained by performing the following steps :

   - preparation of a suspension loaded with calcium phosphate powder and containing optionally at least one other poorly soluble calcium salt,
   - optional addition of at least one inorganic or organic additive,
   - optional addition of at least one gelling agent,
   - passing of the suspension through a nozzle,
   - flowing out of the nozzle in the form of a laminar jet,
   - perturbation of the laminar jet so that it is segmented into small droplets,
   - consolidation of the droplets,

   each microgranule thus formed having roughly the shape of a revolution ellipsoid with major axis a and minor axis b, the granulometric distribution being such that its standard deviation relative to the average length of either of axes **a** or **b** is less than 5 %.

2. Microgranules according to claim 1, **characterised by** the fact that the ratio of axis **a** over axis **b** varies between 1 and 3.

3. Microgranules according to claim 2, **characterised by** the fact that both axes have the same length (ratio between axes a and b equals 1).

4. Microgranules according to claim 3, **characterised by** the fact that the standard deviation relative to the average length of the axes is less than 2 %.

5. Microgranules according to any of the previous claims, **characterised by** the fact that they show a uniformly distributed porosity representing a volumetric fraction comprised between 0 and 70 % of their total apparent volume.

6. Production process of calcium phosphate microgranules of quasi identical volume, **characterised by** the following steps :

   - preparation of a suspension loaded with calcium phosphate powder and containing optionally at least one other poorly soluble calcium salt,
   - optional addition of at least one inorganic or organic additive,
   - optional addition of at least one gelling agent,
   - passing of the suspension through a nozzle,
   - flowing out of the nozzle in the form of a laminar jet,
   - perturbation of the laminar jet so that it is segmented into small droplets,
   - consolidation of the droplets.

**FIG. 1**

petit axe

grand axe

## FIG. 2

Acc.V Spot Magn    Det  WD  Exp  ⊢————————⊣  100 µm
3.00 kV 3.0  200x    SE   28.3 194